# EUROPEAN PATENT APPLICATION

(11) **EP 4 663 235 A1**
(43) Date of publication of application: **17.12.2025**
(21) Application number: 24181280.9
(22) Date of filing: 11.06.2024
(51) Int. Cl.: A61N 1/372, A61B 5/103, A61N 1/36, A61N 1/365, A61N 1/08, A61N 1/37

(54) **IMPLANTABLE MEDICAL DEVICE, METHOD FOR ACTIVATING AN IMPLANTABLE MEDICAL DEVICE, AND METHOD FOR IMPLANTING AN IMPLANTABLE MEDICAL DEVICE**

(71) Applicant: BIOTRONIK SE & Co. KG, 12359 Berlin (DE)
(72) Inventor: Whittington, R. Hollis, Portland, 97202 (US)
(74) Representative: Biotronik Corporate Services SE

(57) **Abstract**

An implantable medical device (IMD) (20) is provided. The IMD (20) comprises: a motion sensor (24) configured for detecting a motion of the implantable medical device (20) and for generating a first motion signal depending on the detected motion; a memory (26) being configured for storing a predetermined first motion signature, wherein the predetermined first motion signature is representative for an implanting motion, which is carried out when implanting the implantable medical device (20); and a controller (22) operatively coupled to the motion sensor (24) and the memory (26), wherein the controller (22) is configured to: receive a first motion signal from the motion sensor (24), receive a first motion signature signal from the memory (26), wherein the first motion signature signal is representative for the predetermined first motion signature, analyze the first motion signal with respect to the predetermined first motion signature, and switch the implantable medical device (20) from a storage mode to an active mode, if at least a part of the first motion signal fulfils a predetermined first criterion related to the first motion signature.

## Description

The present invention relates to an implantable medical device, to a method for activating the implantable medical device, and to a method for implanting the implantable medical device.

Implantable medical devices (IMDs) are becoming increasingly autonomous and connected via wireless communication to the outside world. In several applications it becomes increasingly important to minimize or optimize workflows surrounding an implantation of the IMDs into the corresponding body. IMDs such as implantable cardiac monitors (ICMs), implantable pressure sensors (IPS) and implantable leadless pacemakers (II,Ps) enable to reduce, ideally to minimize the time needed for the implantation in an operating room. The ICMs are tools used for diagnosis of unknown arrhythmias, palpitations, identification of atrial fibrillation (AF), and other arrhythmias.

The implantation of the ICMs, for example, is more frequently carried out outside the operating room, in particular in procedure rooms and even doctor's offices. In such settings, which may be referred to as "Office Based Labs (OBLs)", it may be common that a programmer and/or a programmer unit for the corresponding ICM is not present. Therefore, it may be important that the IMDs, in particular the ICMs, provide a possibility to be activated from its storage mode, in other words "shelf state", without the use of additional tools or electronics.

Conventional ILPs may be activated before being implanted. Alternatively, an activation of conventional ILPs within the body may be carried out by using one or more telemetry coils, wherein this process also requires the presence of a programmer unit. These programmer units are expensive to have at an OBL. Further, an activation via Radio Frequency (RF) from outside of the body requires the corresponding IMD to listening for a corresponding RF signal. This consumes power and limits a shelf lifetime of the IMDs, and ultimately the longevity of the IMDs, which is an important factor for competitiveness and for achieving the longest possible monitoring. Therefore, e.g. for the ILPs, it would be convenient, if the ILPs may activate their pacing or sensing modes automatically, in particular upon implantation. In this case, it would not be required to communicate with the ILP using a coil for activating the corresponding ILP.

It is an object of the present invention to provide an implantable medical device, which provides the possibility of automatically and safely being activated upon implantation into the corresponding body, in particular without the need for an additional hardware or device, i.e. autonomously.

It is another object of the present invention to provide a method for activating the implantable medical device, which provides the possibility of automatically and safely activating the implantable medical device upon implantation into the corresponding body, in particular without the need for any additional hardware or device, i.e. autonomously.

Such objects may be met with the subject-matter of the independent claims. Advantageous embodiments are defined in the dependent claims as well as the corresponding specification and figures.

According to a first aspect of the present invention, an implantable medical device (IMD) is provided. The IMD comprises: a motion sensor configured for detecting a motion of the IMD and for generating a first motion signal depending on the detected motion; a memory being configured for storing a predetermined first motion signature, wherein the predetermined first motion signature is representative for an implanting motion, which is typically carried out when implanting the IMD, in particular into a body, e.g. into a human body; and a controller operatively coupled to the motion sensor and the memory, wherein the controller is configured to: receive a first motion signal from the motion sensor, receive a first motion signature signal from the memory, wherein the first motion signature signal is representative for the predetermined first motion signature, analyse the first motion signal with respect to the predetermined first motion signature, and switch the IMD from a storage mode to an active mode, if at least a part of the first motion signal fulfils a predetermined first criterion related to the first motion signature.

According to a second aspect of the present invention, a method for activating the IMD in the corresponding body, wherein the IMD comprises the motion sensor configured for detecting the first motion of the IMD and for generating the first motion signal depending on the detected motion, the memory being configured for storing the predetermined first motion signature, wherein the predetermined first motion signature is representative for the implanting motion, which is typically carried out when implanting the IMD, and the controller operatively coupled to the motion sensor and the memory, the method comprising: receiving, by the controller, the first motion signal from the motion sensor, receiving, by the controller, the first motion signature signal from the memory, wherein the first motion signature signal is representative for the predetermined first motion signature, analysing, by the controller, the first motion signal with respect to the predetermined first motion signature, and switching, by the controller, the IMD from the storage mode to the active mode, if at least the part of the first motion signal fulfils the predetermined first criterion related to the first motion signature.

According to a third aspect of the present invention, a method for implanting the IMD into the corresponding body is provided. The method comprises moving, e.g. pushing, the IMD under a skin of the body and activating the IMD in accordance with the above method for activating the IMD.

Briefly summarised in a non-limiting manner, embodiments of the present invention relate to IMDs which provide the possibility of being activated automatically and without the need for any additional hardware and to the corresponding method for automatically activating the corresponding IMDs. The present invention is based on the insight that a motion of an IMD, which is typically carried out when the IMD is implanted, has a very characteristic signature which may be easily distinguished from a normal handling of the IMD outside of the body. This signature may be safely recognized, wherein the recognition of the implanting motion having the signature may be used for activating the corresponding IMD automatically and autonomically.

The above solution facilitates the activation the IMDs inside the body. Several advantages result from the above construction: The IMDs provide the possibility to be automatically activated from its storage mode without the use of additional tools or electronics, i.e. automatically and autonomously. The IMDs do not have to be activated before being implanted. No telemetry coils and/or programmer unit are necessary for the activation. No activation via Radio Frequency from outside of the body is required, contributing to low energy needs, thereby to a long shelf lifetime, and ultimately to the longevity of the corresponding IMD. This enhances the competitiveness and may achieve the longest possible monitoring.

Analyzing the first motion signal with respect to the predetermined first motion signature contributes to that the implanting procedure, which involves the implanting motion, is securely recognized. If the first motion signal or at least the part of the first motion signal does not fulfil the predetermined first criterion related to the first motion signature, the IMD is not switched from the storage mode to the active mode. Switching the IMD from the storage mode to the active mode, if at least a part of the first motion signal is representative for the predetermined first motion signature, contributes to that the IMD is not activated before being implanted and that the IMD is securely activated when being implanted. This enables to automatically and safely activate the IMD within the corresponding body without any additional resources as for example a device and/or a person for activating the IMD. Using the motion sensor for detecting the implanting procedure may contribute to save energy before activating the IMD, because there are several appropriate motion sensors known, which provide a storage mode which consumes very little energy. The first motion signature may be determined in advance, e.g. when implanting a representative IMD of the same type, e.g. in the human body, and may be predetermined for carrying out the above method.

In the storage mode of the IMD, the motion sensor and the controller may also be in a storage mode. That may mean that only those components of the motion sensor and of the controller are active in the storage mode, which are necessary to gather the first motion of the IMD and to analyze the first motion signal with respect to the predetermined first motion signature. In the active mode of the IMD at least the communication module is activated, preferably the IMD is ready for its intended purpose in the human body.

In the following, characteristics of embodiments of the present invention will be described in more detail.

According to an embodiment, the predetermined first motion signature comprises one or more predetermined first motion parameter thresholds, and the first motion signal fulfils the predetermined first criterion related to the first motion signature, if one or more motion parameter values encoded in the first motion signal pass the corresponding predetermined first motion parameter thresholds. Using the motion parameter values as a decision support for deciding whether to switch the IMD on, in other words to activate it or not, may contribute to save energy of the IMD before activating the IMD, because checking whether the motion parameter values exceed the corresponding predetermined first motion parameter thresholds may be carried out by using a small amount of energy only.

For example, one possible motion parameter may refer to an amplitude of a motion along one single axis of the IMD, which corresponds to an implanting direction of the IMD. Alternatively or additionally, one motion parameter may refer to an amplitude of a motion perpendicular to the one single axis of the IMD. That one of the motion parameter values pass the corresponding predetermined first motion parameter thresholds may mean that the corresponding motion parameter value exceeds or undercuts the corresponding predetermined first motion parameter thresholds depending on the corresponding parameter. For example, if the parameter refers to the amplitude of the motion along the single axis of the IMD, which corresponds to the implanting direction of the IMD, an exceeding of the corresponding predetermined first motion parameter threshold may be representative for the implanting procedure. In contrast, if the parameter refers to the amplitude of the motion perpendicular to the single axis of the IMD, an undercutting of the corresponding predetermined first motion parameter threshold may be representative for the implanting procedure.

According to an embodiment, the motion parameter, to which one of the motion parameter values may refer, may refer to the amplitude of the motion of the IMD in one single direction, e.g. in a direction parallel to a longitudinal axis of the IMD. Alternatively or additionally, another motion parameter, to which another one of the parameter values may refer, may refer to an acceleration of the implantable medical device in the one single direction. Alternatively or additionally, another motion parameter, to which another one of the parameter values may refer, may refers to a speed of the implantable medical device in the one single direction. Using the amplitude, the acceleration, and/or the speed of the first motion as a decision support for deciding whether to switch the IMD on, in other words to activate it, or not may contribute to save energy of the IMD before activating the IMD, because checking whether the amplitude, the acceleration, and/or the speed of the first motion exceed or undercut the corresponding predetermined first motion parameter thresholds, in particular a predetermined amplitude threshold, a predetermined acceleration threshold, and/or a predetermined speed threshold, may be carried out by using a small amount of energy only. The movement, acceleration, and/or speed of the implantable medical device in one single direction may also be referred to as movement, acceleration, and/or, respectively, speed along a single axis of the implantable medical device or as motion exclusively in one single direction or along the one single axis. This single direction and/or single axis may be parallel to a longitudinal axis of the implantable medical device.

According to an embodiment, the predetermined first motion signature comprises a predetermined first motion pattern which corresponds to a recorded first motion signal or at least a part of the recorded first motion signal, which was generated by a motion sensor of the same kind when the corresponding implantable medical device was implanted, and at least the part of the first motion signal fulfils the predetermined first criterion related to the first motion signature, if at least the part of the first motion signal corresponds to the predetermined first motion pattern or if a difference between the part of the first motion signal and the predetermined first motion pattern is smaller than a predetermined first motion pattern threshold. Comparing the first motion signal with the predetermined first motion pattern, which was generated by the motion sensor of the same kind when the corresponding implantable medical device was implanted, may contribute to that the implanting procedure is detected very reliably. The first motion pattern may be recognized and/or extracted from the recorded first motion signal by a neural network, which was trained on recognizing signal patterns in signals. The motion sensor of the same kind may be the same motion sensor which is arranged in the IMD, or another motion sensor of the same kind which is arranged in another IMD of the same type. The difference may be determined by any known mathematical method for comparing electric signals and for calculating a difference between these signals. Optionally, the determining whether at least the part of the first motion signal corresponds to the predetermined first motion pattern or whether the difference between the part of the first motion signal and the predetermined first motion pattern is smaller than the predetermined first motion pattern threshold may be carried out by a neural network running within the controller and being trained for comparing electrical signals.

According to an embodiment, the IMD comprises at least one further sensor configured for sensing at least one body signal from the body in which the IMD is implanted and for generating a sensor signal which is representative for the body signal, wherein the controller is configured to: receive the sensor signal from the further sensor, switch the IMD from the storage mode to the active mode only, if the sensor signal and/or the corresponding body signal and/or a body parameter value encoded in the sensor signal fulfils a predetermined second criterion. Using the further sensor(s) for checking whether the IMD is really implanted may increase a reliability of the recognition of the implanting procedure by the IMD. Detecting the body signal of the body and checking whether the corresponding sensor signal, body signal, and/or, respectively, body parameter value fulfils the predetermined second criterion may enable to double-check whether the IMD really is implanted in the corresponding body. In this context, in case of the sensor signal, the predetermined second criterion may be that a difference between a predetermined sensor signal pattern and at least a part of the sensor signal is smaller than a predetermined sensor signal threshold. In case of the body signal, the predetermined second criterion may be that a difference between a predetermined body signal pattern and at least a part of the body signal is smaller than a predetermined body signal threshold. In case of the body parameter value, the predetermined second criterion may be that the body parameter value passes a predetermined body parameter value threshold.

According to an embodiment, one further sensor of the above mentioned further sensors is a temperature sensor, and the body signal is representative for a temperature of the body. In this case, the parameter value may be the temperature of the body, the predetermined parameter value threshold may be a predetermined temperature threshold and the predetermined second criterion may be fulfilled when the temperature exceeds the predetermined temperature threshold. Alternatively, the predetermined second criterion may refer to a temperature interval in which the temperature must reside in order to fulfil the predetermined criterion. The temperature interval may be e.g. 35° to 38° C, e.g. 36° to 37° C. Alternatively, the predetermined second criterion may refer to a row of subsequently measured temperatures and the predetermined criterion may be fulfilled when the temperature arrives at a normal body temperature of the corresponding body in a predetermined duration. The temperature sensor may be one of the further sensors mentioned in context of the preceding embodiments.

According to an embodiment, one further sensor of the above mentioned further sensors is an electric sensor for sensing an electric activity in the body, and the electric activity is representative for a heartbeat of a heart of the body. In this case, the parameter value may be a voltage, a current, or an impedance between electrodes of the IMD measured in the body, the predetermined parameter value threshold may be a predetermined voltage threshold, a predetermined current threshold, or, respectively, a predetermined impedance threshold, and the predetermined second criterion may be fulfilled when the voltage passes the predetermined voltage threshold, when the current passes the predetermined current threshold, or, respectively, when the impedance passes the predetermined impedance threshold. Alternatively, in this case, the body signal may be representative for an ElectroCardioGram (ECG) and the body signal may fulfil the predetermined criterion, if the ECG encoded in the body signal corresponds or at least nearly corresponds to a predetermined ECG pattern. In particular, a pacing and sensing behaviour could be observed from the corresponding patient's rhythm using ECG. In this way, a therapy could be confirmed without the need for immediate interaction with additional hardware and, in case, a corresponding programmer. The electric sensor may be a voltage sensor or a current sensor. The electric sensor may be one of the further sensors mentioned in context of the pre-preceding embodiment.

According to an embodiment, the memory is configured for storing a predetermined second motion signature, wherein the predetermined second motion signature is representative for a typical motion within the corresponding body, wherein the controller is configured to: receive a second motion signal from the motion sensor, receive a second motion signature signal from the memory, wherein the second motion signature signal is representative for the predetermined second motion signature, analyse the second motion signal with respect to the predetermined second motion signature, and switch the implantable medical device from the storage mode to the active mode only, if at least a part of the second motion signal fulfils a predetermined third criterion related to the second motion signature. Detecting the typical motion of the body by analyzing the second motion signal with respect to the predetermined second motion signature may enable to double-check whether the IMD really is implanted in the corresponding body. The typical motion of the body may be a heartbeat or a respiration movement of the body in which the implantable medical device is implanted.

According to an embodiment, the IMD is configured as implantable monitor, implantable pressure sensor or as implantable leadless pacemaker. The implantable monitor may for example be an Implantable Cardiac Monitor.

According to an embodiment, the implantable medical device comprises the at least one further sensor configured for sensing the at least one body signal from the body in which the implantable medical device is implanted and for generating the sensor signal which is representative for the body signal, wherein the method further comprises: receiving, by the controller, the sensor signal from the further sensor, analysing, by the controller, the sensor signal with respect to the body signal, and switching, by the controller, the implantable medical device from the storage mode to the active mode only, if the body signal or a parameter value encoded in the body signal fulfils a predetermined criterion.

Alternatively or additionally, the memory is configured for storing the predetermined second motion signature, wherein the predetermined second motion signature is representative for the typical motion within the corresponding body. In this case, the method may further comprise: receiving the second motion signal from the motion sensor, receiving the second motion signature signal from the memory, wherein the second motion signature signal is representative for the predetermined second motion signature, analysing the second motion signal with respect to the predetermined second motion signature, and switching the implantable medical device from the storage mode to the active mode only, if at least a part of the second motion signal is representative for the predetermined second motion signature.

It shall be noted that possible features and advantages of embodiments of the invention are described herein with respect to various embodiments of the IMD, on the one hand, and with respect to various embodiments of the method for activating the IMD, on the other hand, as well as with respect to method for implanting the IMD. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawings. However, neither the drawings nor the description shall be interpreted as limiting the invention.
- Fig. 1: shows an exemplary embodiment of an implantable medical device.
- Fig. 2: shows a flowchart of an exemplary embodiment of a method for activating the implantable medical device.
- Fig. 3: shows a flowchart of an exemplary embodiment of a method for verifying an implantation of the implantable medical device.
- Fig. 4: shows a flowchart of an exemplary embodiment of a method for verifying an implantation of the implantable medical device.

The figures are only schematic and not to scale. Same reference signs refer to same or similar features.

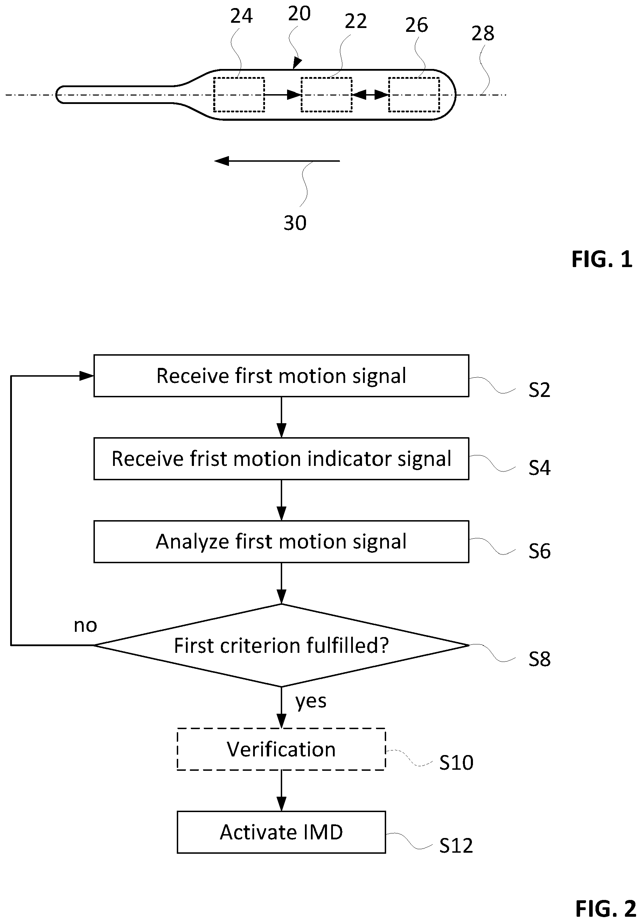

Fig. 1 shows an exemplary embodiment of an implantable medical device (IMD) 20. The IMD may be configured as implantable monitor, as implantable pressure sensor, or as leadless pacemaker (ILP). The implantable monitor may for example be an Implantable Cardiac Monitor (ICM). The IMD 20 comprises a motion sensor 24, a memory 26 and a controller 22. The IMD 20 may comprises one or more further sensors (not shown), e.g. a temperature sensor, a voltage sensor, a current sensor, and/or an impedance sensor. The IMD 20 provides the possibility of being activated automatically and without the need for any additional hardware.

A motion of the IMD 20, which is typically carried out when the IMD 20 is implanted, has a very characteristic signature. This signature may be safely recognized, wherein the recognition of the implanting motion having the signature may be used for activating the IMD 20 automatically and autonomically. For example, the IMD 20 comprises a longitudinal axis 28. The longitudinal axis 28 may be a symmetry axis of the IMD 20. In this case, the implanting motion may be a motion of the IMD 20 parallel to the longitudinal axis only, i.e. not in any other direction. Further, this implanting motion may have a given acceleration and/or speed profile, depending on the implanting tool used for implanting the IMD 20. In fact, a given implanting tool may provide the same acceleration and/or speed profile for any IMD 20 when being implanted with this implanting tool.

The motion sensor 24 is configured for detecting the motion of the IMD 20 and for generating a first motion signal depending on the detected motion. The motion sensor 24 may be a multi-axis motion sensor, e.g. a 3-axis motion sensor.

The memory 26 is configured for storing a predetermined first motion signature, wherein the predetermined first motion signature is representative for the implanting motion, which is carried out when implanting the IMD 20, in particular into a body, e.g. into a human body.

The controller 22 is operatively coupled to the motion sensor 24 and the memory 26. The controller 22 is configured to receive a first motion signal from the motion sensor 24, to receive a first motion signature signal from the memory 26, wherein the first motion signature signal is representative for the predetermined first motion signature, to analyse the first motion signal with respect to the predetermined first motion signature, and to switch the IMD 20 from a storage mode to an active mode, if at least a part of the first motion signal fulfils a predetermined first criterion related to the first motion signature.

The predetermined first motion signature may comprise one or more predetermined first motion parameter thresholds, and the first motion signal may fulfil the predetermined first criterion related to the first motion signature, if one or more motion parameter values encoded in the first motion signal pass the corresponding predetermined first motion parameter thresholds. For example, one possible motion parameter may refer to an amplitude of a motion along the longitudinal axis 28 of the IMD 20, which corresponds to an implanting direction of the IMD 20. Alternatively or additionally, one motion parameter may refer to an amplitude of a motion perpendicular to the longitudinal axis 28 of the IMD.

That one of the motion parameter values pass the corresponding predetermined first motion parameter thresholds may mean that the corresponding motion parameter value exceeds or undercuts the corresponding predetermined first motion parameter thresholds depending on the corresponding parameter. For example, if the parameter refers to the amplitude of the motion along the longitudinal axis 28 of the IMD 20, which corresponds to the implanting direction of the IMD 20, an exceeding of the corresponding predetermined first motion parameter threshold may be representative for the implanting procedure. In contrast, if the parameter refers to the amplitude of the motion perpendicular to the longitudinal axis 28 of the IMD 20, an undercutting of the corresponding predetermined first motion parameter threshold may be representative for the implanting procedure.

So, the motion parameter, to which one of the motion parameter values may refer, may refer to the amplitude of the motion of the IMD 20 in the longitudinal direction only, wherein the longitudinal direction may be parallel to the longitudinal axis 28. Alternatively or additionally, another motion parameter, to which another one of the parameter values may refer, may refer to an acceleration of the implantable medical device in the longitudinal direction. Alternatively or additionally, another motion parameter, to which another one of the parameter values may refer, may refer to a speed of the implantable medical device in the longitudinal direction. The movement, acceleration, and/or speed of the IMD 20 in the longitudinal direction may also be referred to as movement, acceleration, and/or, respectively, speed along the longitudinal axis 28 of the IMD 20 or as motion exclusively in the longitudinal direction or along the longitudinal axis 28.

Alternatively, instead of the one or more predetermined first motion parameter thresholds, the predetermined first motion signature may comprise a predetermined first motion pattern which corresponds to a recorded first motion signal or at least a part of the recorded first motion signal, which was generated by a motion sensor of the same kind when the corresponding IMD 20 was implanted, and at least the part of the first motion signal fulfils the predetermined first criterion related to the first motion signature, if at least the part of the first motion signal corresponds to the predetermined first motion pattern or if a difference between the part of the first motion signal and the predetermined first motion pattern is smaller than a predetermined first motion pattern threshold. Comparing the first motion signal with the predetermined first motion pattern, which was generated by the motion sensor of the same kind when the corresponding IMD 20 was implanted, may contribute to that the implanting procedure is detected very reliably.

The first motion signature may be recognized and/or extracted from the recorded first motion signal by a neural network, which was trained on recognizing signal patterns in signals. The motion sensor of the same kind may be the same motion sensor 24 which is arranged in the IMD 20, or another motion sensor of the same kind which is arranged in another IMD of the same type. The difference may be determined by any known mathematical method for comparing electric signals and for calculating a difference between these signals, e.g. by a mathematical difference metric. Optionally, the determining whether at least the part of the first motion signal corresponds to the predetermined first motion pattern or whether the difference between the part of the first motion signal and the predetermined first motion pattern is smaller than the predetermined first motion pattern threshold may be carried out by a neural network running within the controller.

Optionally, the IMD 20 may comprise at least one of the further sensors (not shown) configured for sensing at least one body signal from the body in which the IMD 20 is implanted and for generating a sensor signal which is representative for the body signal. In this context, the controller 22 may be configured to: receive the sensor signal from the further sensor, and to switch the IMD 20 from the storage mode to the active mode only, if the sensor signal and/or the corresponding body signal and/or a body parameter value encoded in the sensor signal fulfils a predetermined second criterion. Using the further sensor(s) for checking whether the IMD 20 is really implanted within the body may increase a reliability of the recognition of the implanting procedure by the IMD 20. Detecting the body signal of the body and checking whether the corresponding sensor signal, body signal, and/or, respectively, body parameter value fulfils the predetermined second criterion may enable to double-check whether the IMD 20 really is implanted in the corresponding body. In this context, in case of the sensor signal, the predetermined second criterion may be that a difference between a predetermined sensor signal signature and at least a part of the sensor signal is smaller than a predetermined sensor signal threshold. In case of the body signal, the predetermined second criterion may be that a difference between a predetermined body signal signature and at least a part of the body signal is smaller than a predetermined body signal threshold. In case of the body parameter value, the predetermined second criterion may be that the body parameter value passes a predetermined body parameter value threshold.

For example, one of the further sensor(s) may be a temperature sensor. In this context, the body signal may be representative for a temperature of the body. In this case, the parameter value may be the temperature of the body, the predetermined parameter value threshold may be a predetermined temperature threshold. Then, the predetermined second criterion may be fulfilled when the temperature exceeds the predetermined temperature threshold. Alternatively, the predetermined second criterion may refer to a temperature interval in which the temperature must reside in order to fulfil the predetermined second criterion. Alternatively, the predetermined second criterion may refer to a row of subsequently measured temperatures and the predetermined second criterion may be fulfilled when the temperature arrives at a normal body temperature of the corresponding body in a predetermined duration.

Alternatively or additionally, one of the further sensor(s) may be an electric sensor for sensing an electric activity in the body, wherein the electric activity may be representative for a heartbeat of a heart of the body. In this case, the parameter value may be a voltage or a current measured in the body, the predetermined parameter value threshold may be a predetermined voltage threshold or, respectively, a predetermined current threshold, and the predetermined second criterion may be fulfilled when the voltage passes the predetermined voltage threshold or, respectively, when the current passes the predetermined current threshold. Alternatively, in this case, the body signal may be representative for an ElectroCardioGram (ECG) and the body signal may fulfil the predetermined criterion, if the ECG encoded in the body signal corresponds or at least nearly corresponds to a predetermined ECG. In particular, a pacing and sensing behaviour could be observed from the corresponding patient's rhythm using ECG. In this way, a therapy could be confirmed without the need for immediate interaction with additional hardware and, in case, a corresponding programmer. The electric sensor may be a voltage sensor or a current sensor.

Optionally, the memory 26 may be configured for storing a predetermined second motion signature, wherein the predetermined second motion signature may be representative for a typical motion within the corresponding body and/or of the corresponding body. In this context, the controller 22 may be configured to: receive a second motion signal from the motion sensor 24, receive a second motion signature signal from the memory 26, wherein the second motion signature signal is representative for the predetermined second motion signature, analyse the second motion signal with respect to the predetermined second motion signature, and switch the IMD 20 from the storage mode to the active mode only, if at least a part of the second motion signal fulfils a predetermined third criterion related to the second motion signature. The typical motion of the body may be a heartbeat or a respiration movement of the body in which the IMD 20 is implanted.

Fig. 2 shows a flowchart of an exemplary embodiment of a method for activating the IMD 20. The method for activating the IMD 20 provides the possibility of activating the IMD 20 automatically and without the need for any additional hardware.

In a step S2, the first motion signal may be received from the motion sensor, e.g. by the controller 22.

In a step S4, the first motion signature signal may be received from the memory 26, e.g. by the controller 22. The first motion signature signal is representative for the predetermined first motion signature, as explained above.

In a step S6, the first motion signal may be analysed with respect to the predetermined first motion signature, e.g. by the controller 22, as explained above.

In a step S8, it is determined whether at least the part of the first motion signal fulfils the predetermined first criterion related to the first motion signature, as explained above with respect to figure 1. If the requirement of step S8 is fulfilled, the method may proceed with optional step S10 or with step S12. If the requirement of step S8 is not fulfilled, the method may proceed with step S2. Optionally, step S8 may be carried out when carrying out step S6. In other words, the analysing of the first motion signal, which is done in step S6, may involve the determination whether the first criterion is fulfilled or not. In some embodiments, the determination whether the first criterion is fulfilled or not may correspond to analysing the first motion signal.

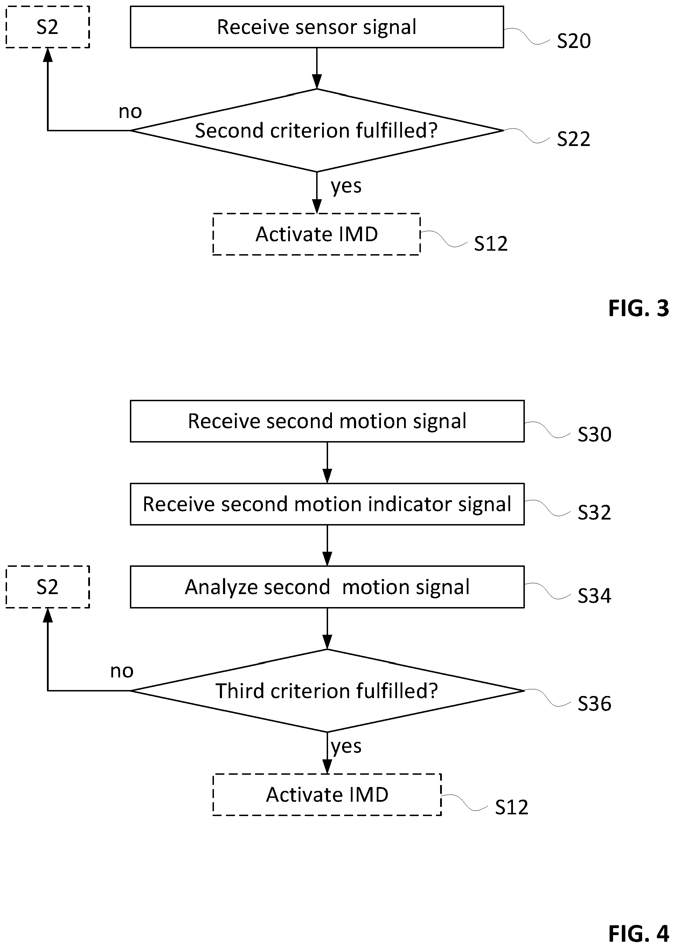

In the optional step S10, it may be verified whether the IMD 20 was really implanted. This verification may be carried out by the method described with respect to figure 3 and/or by the method described with respect to figure 4, as explained below. In case of carrying out the verification by the method described with respect to figure 3 and by the method described with respect to figure 4, the IMD 20 may be activated only if the second and third criterion are fulfilled, or if the second or the third criterion are fulfilled.

In step S12, the IMD 20 may be switched from the storage mode to the active mode, e.g. by the controller 22.

Optionally, when all criteria for activating the IMD 20 are fulfilled, the IMD 20 may not be activated immediately or may not be activated completely immediately. For example, a time period may be defined in advance after which the IMD 20 may be activated or, respectively completely activated, after all criteria for activating the IMD 20 are fulfilled. This may contribute to avoid artifacts associated with the implantation itself. Alternatively or additionally, e.g. in case of the IMD 20 being configured as ICM, the IMD 20 may not record any body signals, e.g. arrhythmias, directly after the implantation, e.g. for one or two hours, to allow the corresponding wound of the body to be closed and properly bandaged after implanting the IMD 20 to avoid artifacts and false detections. Alternatively or additionally, e.g. in case of the IMD 20 being configured as ILP, a relatively high pacemaker pulse amplitude may be used by the IMD 20 until 48 hours after the implantation, and then it is reduced to a relative low pacemaker pulse amplitude, i.e. lower than the high pacemaker pulse, once an inflammation from the implantation may be reduced.

Fig. 3 shows a flowchart of an exemplary embodiment of a method for verifying the implantation of the IMD 20.

In a step S20, the sensor signal from one or more of the further sensors may be received, e.g. by the controller 22.

In a step S22, the sensor signal(s) may be analysed with respect to corresponding body signal encoded in the sensor signal(s), as explained above. In particular, the sensor signal(s) may be analysed with respect to corresponding body signal(s) by determining whether the sensor signal(s) or at least the part of the sensor signal(s) fulfils the predetermined second criterion or not. If the requirement of step S22 is fulfilled, the method may proceed with above step S12, in which the IMD 20 may be activated. If the requirement of step S22 is not fulfilled, the method may proceed with above step S2, may keeping the IMD 20 in the storage mode.

So, according to this embodiment the IMD 20 may be switched from the storage mode to the active mode only, if the body signal or a parameter value encoded in the body signal fulfils the predetermined second criterion.

Fig. 4 shows a flowchart of an exemplary embodiment of a method for verifying an implantation of the IMD 20.

In a step S30 the second motion signal from the motion sensor 24 may be received, e.g. by the controller 22.

In a step S32, the second motion signature signal may be received from the memory 26, e.g. by the controller 22. The second motion signature signal may be representative for the predetermined second motion signature, as explained above.

In a step S34, the second motion signal may be analysed with respect to the predetermined second motion signature. Analysing the second motion signal with respect to the predetermined second motion signature may involve a step S36, in which it is determined whether the second motion signal fulfills the third criterion, as explained above. If the requirement of step S36 is fulfilled, the method may proceed with above step S12, in which the IMD 20 is activated. If the requirement of step S36 is not fulfilled, the method may proceed with above step S2, keeping the IMD 20 in the storage mode.

A method for implanting the IMD 20 into the corresponding body may comprise moving, e.g. pushing, the IMD 20 under the skin of the corresponding body, e.g. in correspondence with the implanting motion, and activating the IMD 20 in accordance with the above method for activating the IMD 20. The IMD 20 may be arranged in an implanting tool (not shown) for implanting the IMD 20 into the body. The IMD 20 may be implanted catheter based.

The invention is not restricted to the above embodiments. For example, the above embodiments may be combined. For example, the method for activating the IMD 20 may not comprise the verification step S10. Further, the IMD 20 may comprise the one or more further sensors. Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of Reference Numerals

- 20: implantable medical device
- 22: controller
- 24: motion sensor
- 26: memory
- 28: longitudinal axis
- 30: implanting direction
- S2-S36: steps two to thirty-six

## Claims

1. An implantable medical device (20), comprising:
a motion sensor (24) configured for detecting a motion of the implantable medical device (20) and for generating a first motion signal depending on the detected motion;
a memory (26) being configured for storing a predetermined first motion signature, wherein the predetermined first motion signature is representative for an implanting motion, which is typically carried out when implanting the implantable medical device (20); and
a controller (22) operatively coupled to the motion sensor (24) and the memory (26), wherein the controller (22) is configured to:
receive a first motion signal from the motion sensor (24),
receive a first motion signature signal from the memory (26), wherein the first motion signature signal is representative for the predetermined first motion signature,
analyze the first motion signal with respect to the predetermined first motion signature, and
switch the implantable medical device (20) from a storage mode to an active mode, if at least a part of the first motion signal fulfils a predetermined first criterion related to the first motion signature.

2. The implantable medical device (20) of claim 1, wherein
the predetermined first motion signature comprises one or more predetermined first motion parameter thresholds, and
at least the part of the first motion signal fulfils the predetermined first criterion related to the first motion signature, if one or more motion parameter values encoded in the first motion signal pass the corresponding predetermined first motion parameter thresholds.

3. The implantable medical device (20) of claim 2, wherein
a motion parameter, to which one of the motion parameter values refers, refers to an amplitude of a motion of the implantable medical device (20) in one single direction,
another motion parameter, to which another one of the parameter values refers, refers to an acceleration of the implantable medical device (20) in the one single direction, and/or
another parameter, to which another one of the parameter values refers, refers to a speed of the implantable medical device (20) in one single direction.

4. The implantable medical device (20) of claim 1, wherein
the predetermined first motion signature comprises a predetermined first motion pattern which corresponds to a recorded first motion signal or at least a part of the recorded first motion signal, which was generated by a motion sensor (24) of the same kind when the corresponding implantable medical device (20) was implanted, and
at least the part of the first motion signal fulfils the predetermined first criterion related to the first motion signature, if at least the part of the first motion signal corresponds to the predetermined first motion pattern or if a difference between the part of the first motion signal and the predetermined first motion pattern is smaller than a predetermined first motion pattern threshold.

5. The implantable medical device (20) of any one of the preceding claims, comprising:
at least one further sensor configured for sensing at least one body signal from the body in which the implantable medical device (20) is implanted and for generating a sensor signal which is representative for the body signal, wherein the controller (22) is configured to:
receive the sensor signal from the further sensor, and
switch the implantable medical device (20) from the storage mode to the active mode only, if the sensor signal and/or the corresponding body signal and/or a body parameter value encoded in the sensor signal fulfils a predetermined second criterion.

6. The implantable medical device (20) of claim 5, wherein
one further sensor is a temperature sensor, and
the body signal is representative for a temperature of the body.

7. The implantable medical device (20) of any one of claims 5 or 6, wherein
one further sensor is an electric sensor for sensing an electric activity in the body, and
the electric activity is representative for a heartbeat of a heart of the body.

8. The implantable medical device (20) of any one of the preceding claims, wherein the memory (26) is configured for storing a predetermined second motion signature, wherein the predetermined second motion signature is representative for a typical motion within the corresponding body, wherein the controller (22) is configured to:
receive a second motion signal from the motion sensor (24),
receive a second motion signature signal from the memory (26), wherein the second motion signature signal is representative for the predetermined second motion signature,
analyze the second motion signal with respect to the predetermined second motion signature, and
switch the implantable medical device (20) from the storage mode to the active mode only, if at least a part of the second motion signal fulfils a predetermined third criterion related to the second motion signature.

9. The implantable medical device (20) of any one of the preceding claims, being configured as implantable monitor or as leadless pacemaker.

10. A method for activating an implantable medical device (20) in a body, wherein the implantable medical device (20) comprises a motion sensor (24) configured for detecting a first motion of the implantable medical device (20) and for generating a first motion signal depending on the detected motion, a memory (26) being configured for storing a predetermined first motion signature, wherein the predetermined first motion signature is representative for an implanting motion, which is typically carried out when implanting the implantable medical device (20), and a controller (22) operatively coupled to the motion sensor (24) and the memory (26), the method comprising:
receiving, by the controller (22), a first motion signal from the motion sensor (24),
receiving, by the controller (22), a first motion signature signal from the memory (26), wherein the first motion signature signal is representative for the predetermined first motion signature,
analyzing, by the controller (22), the first motion signal with respect to the predetermined first motion signature, and
switching, by the controller (22), the implantable medical device (20) from a storage mode to an active mode, if at least a part of the first motion signal fulfils a predetermined first criterion related to the first motion signature.

11. The method of claim 10, wherein
the predetermined first motion signature comprises one or more predetermined first motion parameter thresholds, and
the first motion signal fulfils the predetermined first criterion related to the first motion signature, if one or more motion parameter values encoded in the first motion signal pass the corresponding predetermined first motion parameter thresholds.

12. The method of claim 11, wherein
a motion parameter, to which one of the motion parameter values refers, refers to an amplitude of a motion of the implantable medical device (20) in one single direction,
another motion parameter, to which another one of the parameter values refers, refers to an acceleration of the implantable medical device (20) in the one single direction, and/or
another parameter, to which another one of the parameter values refers, refers to a speed of the implantable medical device (20) in one single direction.

13. The method of claim 10, wherein
the predetermined first motion signature comprises a predetermined first motion pattern which corresponds to a recorded first motion signal or at least a part of the recorded first motion signal, which was generated by a motion sensor (24) of the same kind when the corresponding implantable medical device (20) was implanted, and
at least the part of the first motion signal is determined as being representative for the predetermined first motion pattern, if at least the part of the first motion signal corresponds to the predetermined first motion pattern or if a difference between the part of the first motion signal and the predetermined first motion pattern is smaller than a predetermined first motion pattern threshold.

14. The method of any one of claims 10 to 13, wherein the implantable medical device (20) comprises at least one further sensor configured for sensing at least one body signal from the body in which the implantable medical device (20) is implanted and for generating a sensor signal which is representative for the body signal, the method further comprising:
receiving, by the controller (22), the sensor signal from the further sensor,
analyzing, by the controller (22), the sensor signal with respect to body signal, and
switching, by the controller (22), the implantable medical device (20) from the storage mode to the active mode only, if the body signal or a parameter value encoded in the body signal fulfils a predetermined second criterion.

15. A method for implanting an implantable medical device (20) according to one of claims 1 to 9 into a body, the method comprising moving the implantable medical device (20) under a skin of the body and activating the implantable medical device (20) in accordance with the method according to one of claims 10 to 14.
